## Europäisches Patentamt
## European Patent Office
## Office européen des brevets

(19)

(11) Veröffentlichungsnummer: **0 281 128**
**A1**

(12) **EUROPÄISCHE PATENTANMELDUNG**

(21) Anmeldenummer: 88103275.9

(22) Anmeldetag: 03.03.88

(51) Int. Cl.⁴: **G01N 33/86** , //C08B11/145, C08B31/12

(30) Priorität: 04.03.87 CS 1443/87

(43) Veröffentlichungstag der Anmeldung:
07.09.88 Patentblatt 88/36

(84) Benannte Vertragsstaaten:
**BE DE FR GB IT NL SE**

(71) Anmelder: **SLOVENSKA AKADEMIA VIED
Obráncov mieru 49
Bratislava(CS)**

(72) Erfinder: **Antal, Miroslav, Dipl.-Ing. CSc
Bratislava
Svetlá 3(CS)**
Erfinder: **Toman, Rudolf, Dipl.-Ing. CSc
Bratislava
Belopotockého 1(CS)**

(74) Vertreter: **Patentanwälte Beetz sen. - Beetz
jun. Timpe - Siegfried - Schmitt-Fumian-
Mayr
Steinsdorfstrasse 10
D-8000 München 22(DE)**

(54) **Verfahren und Präparat zur Abtrennung von Heparin aus Blut in vitro.**

(57) Die Erfindung betrifft ein Verfahren sowie ein Präparat zur Abtrennung von Heparin aus Blut bzw. Blutplasma in vitro. Das Präparat enthält mindestens ein blutunlösliches Stärke-und/oder Cellulosederivat mit daran gebundenen tertiären oder quartären Aminogruppen mit einer bevorzugten Austauschkapazität von 0,3 bis 1,3 mmol/g. Besonders vorteilhafte Derivate sind Imidazolium-N,N'-diyl-bis(2-hydroxy-3-propyl)-celluloseether-chlorid sowie Imidazolium-N,N'-diyl-bis(2-hydroxy-3-propyl)-ether-chlorid von vernetzter Stärke. Das vorzugsweise in Tablettenform vorliegende Präparat enthält bis zu 12 Masseteile mikrokristalline Cellulose pro Masseteil des Stärke-bzw. Cellulosederivats. Das Stärke-bzw. Cellulosederivat besitzt vorzugsweise eine Korngröße von 50 bis 300 μm, jedoch ist es auch möglich, Derivate von kleinerer oder größerer Korngröße zu verwenden. Das Präparat ist in der Medizin und insbesondere in der Hämatologie bei der Bestimmung des realen hämostatischen Potentials verwendbar, ferner überall dort, wo eine einfache und rasche Abtrennung von Heparin aus Blut bw. Blutplasma in vitro gefordert wird.

## Verfahren und Präparat zur Abtrennung von Heparin aus Blut in vitro

Die Erfindung betrifft ein Verfahren sowie ein Präparat zur Abtrennung von Heparin aus Blut und insbesondere aus Blutplasma in vitro.

Unter dem Begriff "Heparin" werden im Rahmen der Erfindung Heparin selbst sowie Heparinfraktionen, Heparinderivate, Heparinfragmente und/oder Heparinoide verstanden.

Heparin wird bekanntlich häufig an Thromboembolien leidenden Patienten oder auch bei verschiedenen Operationen verabreicht. Erfolg bzw. Versagen der Therapie mit Heparin werden ferner durch Hämokoagulationstests, wie den Quick'schen Test sowie etwa die Ermittlung der partiellen Thromboplastin-Plasmazeit, der Rekalzifikations- und der Thrombin-Plasmazeit geprüft. Die erwähnten Tests werden in vitro vorgenommen, wobei, um durch sie das reale hämostatische Potential ermitteln zu können, das applizierte Heparin aus der entnommenen Blut-bzw. Blutplasmaprobe beseitigt werden muß. Häufig wird hierzu das Heparin durch Reaktion mit Protamin neutralisiert (vgl. J.G. Allen et al., J. Lab. Clin. Med 34 (1949) 473, H.A. Perkins et al., J. Lab. Clin. Med. 48 - (1956) 223), jedoch ist eine derartige Titration unsicher, da in den den Patienten entnommenen Blutplasmaproben der Pegel des Heparins zur Zeit der Entnahme nicht bekannt ist. Dies bedeutet, daß ein Protaminüberschuß wie auch nicht neutralisiertes Heparin in der Probe oft nicht nachgewiesen werden, so daß die Ergebnisse der Hämokoagulationstests mit hoher Ungenauigkeit behaftet sind. Auf diesen Umstand wurde bereits in der Fachliteratur hingewiesen (J.S. Wright et al., J. Cardiovas. Surg. 5 (1964) 244). Um diese erwähnten Nachteile zu vermeiden, wurde die Säulenchromatographie zur Beseitigung von Heparin und Protamin aus Blutplasmaproben angewandt (Thompson et al., J. Lab. Clin. Med. 88 (1976) 922). Zur Bindung von bis zu 300 Heparineinheiten aus 1 ml Plasma wurde dabei ECTEOLA-Cellulose eingesetzt, während das Protamin auf Carboxymethylcellulose adsorbiert wurde. Die Verwendung von Ionenaustauschersäulen vor den komplexen Hämokoagulationstests an Plasma ist jedoch mühsam und zeitraubend. Darüber hinaus verbleibt mindestens 1 ml Plasma in den Zwischenräumen der Ionenaustauscher, was insbesondere bei pädiatrischen Untersuchungen, wo nur kleine Plasmamengen für Tests zur Verfügung stehen, einen - schwerwiegenden Nachteil darstellt.

In jüngster Zeit wurden für die Adsorption von Heparin aus Blutplasmaproben Triethylammoniumethylcellulose enthaltende Tabletten benutzt (US-A-4 198 314, US-A-4 226 599). Allerdings treten hierbei Probleme bei der Homogenisierung von Gemischen der angewandten fibrillären Triethylammoniumethylcellulose und granulärer mikrokristalliner Triethyl-Cellulose auf. Aus diesem Grund werden zunächst aus dem Gemisch Preßlinge einer Masse von 650 mg und einer Härte von 12 bis 15 kg hergestellt, die gemahlen werden, worauf das pulverförmige Gemisch auf die geforderte Teilchengröße gesiebt und erst nach gründlichem Vermischen tablettiert wird (US-A-4 198 314, Beispiel 1).

Der Erfindung liegt demgegenüber die Aufgabe zugrunde, unter Vermeidung der oben angeführten Nachteile ein Verfahren sowie ein einfach herstellbares Präparat zur Abtrennung von Heparin aus Blut bzw. Blutplasma anzugeben, mit denen Heparin rasch und zuverlässig aus dem zu untersuchenden Blut bzw. Blutplasma entfernt werden kann, ohne daß negative Nebeneffekte bei anschließenden komplexen Hämokoagulationstests auftreten.

Die Aufgabe wird anspruchsgemäß gelöst. Vorteilhafte Ausführungsformen sind Gegenstand der abhängigen Ansprüche.

Das erfindungsgemäße Präparat zur Abtrennung von Heparin aus Blut und Blutplasma in vitro ist dadurch gekennzeichnet, daß es mindestens ein blutunlösliches Stärkederivat mit daran gebundenen tertiären oder quartären Aminogruppen und/oder mindestens einem Cellulosederivat enthält, das ausgewählt ist unter Diethylaminoethylcellulose, Dimethylaminoethylcellulose, Trimethylammoniummethylcellulose, Diethylaminopropylcellulose, Triethylammoniumpropylcellulose, Dimethylaminopropylcellulose, Trimethylammoniumpropylcellulose, Diethylamino-2-hydroxypropylcellulose, Polyethylenimincelluose, Triethylammonium-2-hydroxypropylcellulose, Dimethylamino-2-hydroxypropylcellulose, mit einem Gemisch von Epichlorhydrin und Diethylamin in alkalischem Medium vernetzter Cellulose, Imidazolium-N,N'-diyl-bis(2-hydroxy-3-propyl)-celluloseether-chlorid, Dimethylamino-bis(2-hydroxy-3-propyl)-celluloseether-chlorid und Benzyldimethylammonium-2-hydroxypropylcellulose.

Die Austauschkapazität beträgt vorzugsweise 0,3 bis 1,3 mmol/g.

Ein besonders günstig geeignetes Cellulosederivat ist Imidazolium-N,N'-diyl-bis(2-hydroxy-3-propyl)-celluloseether-chlorid.

Das Stärkederivat ist vorzugsweise ausgewählt unter
diethylaminoethyl-vernetzter Stärke,
dimethylaminoethyl-vernetzter Stärke,
trimethylammoniumethyl-vernetzter Stärke,
diethylaminopropyl-vernetzter Stärke,
triethylammoniumpropyl-vernetzter Stärke,
dimethylaminopropyl-vernetzter Stärke,
trimethylammoniumpropyl-vernetzter Stärke,
diethylamino-2-hydroxypropyl-vernetzter Stärke,
polyethylenimin-vernetzter Stärke,
triethylammonium-2-hydroxypropyl-vernetzter Stärke,
dimethylamino-2-hydroxypropyl-vernetzter Stärke,
mit einem Gemisch von Epichlorhydrin und Diethylamin in alkalischem Medium vernetzter Stärke,
Imidazolium-N,N'-diyl-bis(2-hydroxy-3-propyl)-etherchlorid von vernetzter Stärke,
Dimethylamino-bis(2-hydroxy-3-propyl)-etherchlorid von vernetzter Stärke
und
benzyldimethylammonium-2-hydroxypropyl-vernetzter Stärke.

Ein besonders günstig geeignetes Stärkederivat ist Imidazolium-N,N-diyl-bis(2-hydroxy-3-propyl)-ether-chlorid von vernetzter Stärke.

Die Teilchengröße des Präparats beträgt vorzugsweise 50 bis 300 μm. Es liegt ferner besonders günstig in Tablettenform vor.

Das erfindungsgemäße Verfahren zur Abtrennung von Heparin aus Blut und Blutplasma in vitro beruht auf dem Inkontaktbringen des Bluts bzw. Blutplasmas mit einem Sorbens für Heparin und der Abtrennung des Bluts bzw. Blutplasmas vom Sorbens und ist dadurch gekennzeichnet, daß als Sorbens ein Präparat wie oben definiert verwendet wird.

Die wesentlichen Vorteile des erfindungsgemäßen Präparats bestehen darin, daß es auf eine einfache und wirtschaftlich vorteilhafte Art gewonnen werden kann und es sich zur raschen Abtrennung von Heparin aus zu untersuchendem Blut bzw. Blutplasma ohne negative Nebeneffekte bei nachfolgenden Hämokoagulationstests eignet.

Die Erfindung wird im folgenden anhand von Ausführungsbeispielen näher erläutert, die sich auf die Herstellung erfindungsgemäßer Präparate sowie ihre Verwendung zur Heparinabtrennung beziehen.

## Beispiel 1

Imidazolium-N,N'-diyl-bis(2-hydroxy-3-propyl)-celluloseether-chlorid (20 g. Korngröße 50 bis 300 μm) mit einer Austauschkapazität von 0,7 mmol/g wurde mit mikrokristalliner, für pharmazeutische Zwecke geeigneter Cellulose (60 g, Korngröße 50 bis 300 μm) gründlich vermischt. Das so erhaltene Gemisch mit guter Fließfähigkeit wurde dann zu Tabletten von je 80 mg Masse und 3 bis 4 kg Festigkeit tablettiert. Jede Tablette enthielt 20 mg Imidazolium-N,N'-diyl-bis(2-hydroxy-3-propyl)-celluloseether-chlorid und 60 mg mikrokristalline Cellulose.

Das Imidazolium-N,N'-diyl-bis(2-hydroxy-3-propyl)-celluloseether-chlorid wurde folgendermaßen hergestellt: Zu 180 g mikrokristalliner Cellulose für pharmazeutische Zwecke wurden 200 ml einer wäßrigen, 17,5 masse-%-igen Natriumhydroxidlösung zugegeben; das Gemisch wurde 30 min bei 20 °C stehengelassen. Dann wurden 110 ml 1,3-Bis(3-chlor-2-hydroxypropyl)-imidazoliniumsulfat (CS-B-229 063) im Gemisch mit 220 ml Wasser zugegeben, worauf das Reaktionsgemisch homogenisiert und 4 h bei 60 °C umgesetzt wurde. Nach Filtrieren wurde das Cellulosederivat mit 1 masse-%-iger Salzsäure und danach mit destilliertem Wasser bis zur negativen Reaktion auf Chloride gewaschen. Anschließend wurde mit Ethanol und Aceton gewaschen und bei 20 °C getrocknet. Schließlich wurde es auf Korngröße von 50 bis 300 μm gesiebt.

## Beispiel 2

Imidazolium-N,N'-diyl-bis(2-hydroxy-3-propyl)-celluloseether-chlorid (50 g, Korngröße 50 bis 300 μm) mit einer Austauschkapazität von 1,3 mmol wurde mit 600 g mikro kristalliner Cellulose für pharmazeutische Zwecke gründlich vermischt. Durch Tablettieren wurden Tabletten von 65 mg Masse und 3 bis 4 kg Festigkeit hergestellt. Jede Tablette enthielt 5 mg Imidazolium-N,N'-diyl-bis(2-hydroxy-3-propyl)-celluloseether-chlorid und 60 mg mikrokristalline Cellulose.

## Beispiel 3

Imidazolium-N,N'-diyl-bis(2-hydroxy-3-propyl)-celluloseether-chlorid aus mikrokristalliner Cellulose (600 g) mit einer Austauschkapazität von 0,3 mmol/g wurde tablettiert, wodurch Tabletten von 120 mg Masse und von 4 bis 5 kg Festigkeit gewonnen wurden. Jede Tablette enthielt 120 mg Imidazolium-N,N'-diyl-bis(2-hydroxy-3-propyl)-celluloseether-chlorid.

## Beispiel 4

Es wurde wie in Beispiel 1 verfahren mit dem Unterschied, daß als Cellulosederivat Diethylaminoethylcellulose verwendet wurde.

**Beispiel 5**

Es wurde wie in Beispiel 2 verfahren mit dem Unterschied, daß als Cellulosederivat Trimethylammoniumethylcellulose verwendet wurde.

**Beispiel 6**

Es wurde wie in Beispiel 1 verfahren mit dem Unterschied, daß als Cellulosederivat Diethylamino-2-hydroxypropylcellulose verwendet wurde.

**Beispiel 7**

Es wurde wie in Beispiel 2 verfahren mit dem Unterschied, daß als Cellulosederivat mit einem Gemisch von Epichlorhydrin und Diethylamin · in einem Alkalimedium modifizierte Cellulose verwendet wurde.

**Beispiel 8**

Es wurde wie in Beispiel 3 verfahren mit dem Unterschied, daß als Cellulosederivat Polyethylenimincellulose verwendet wurde.

**Beispiel 9**

Es wurde wie in Beispiel 1 verfahren mit dem Unterschied, daß als Cellulosederivat Dimethylaminoethylcellulose verwendet wurde.

**Beispiel 10**

Es wurde wie in Beispiel 2 verfahren mit dem Unterschied, daß als Cellulosederivat Triethylammonium-2-hydroxypropylcellulose verwendet wurde.

**Beispiel 11**

Es wurde wie in Beispiel 3 verfahren mit dem Unterschied, daß als Cellulosederivat Trimethylammoniumpropylcellulose verwendet wurde.

**Beispiel 12**

Es wurde wie in Beispiel 1 verfahren mit dem Unterschied, daß als Cellulosederivat Dimethylamino-bis(2-hydroxy-3-propyl)-celluloseether-chlorid verwendet wurde.

**Beispiel 13**

Es wurde wie in Beispiel 3 verfahren mit dem Unterschied, daß als Cellulosederivat Benzyldimethylammonium-2-hydroxy-propyl-cellulose verwendet wurde.

**Beispiel 14**

Es wurde wie in Beispiel 1 verfahren mit dem Unterschied, daß ein Gemisch von Imidazolium-N,N'-diyl-bis(2-hydroxy-3-propyl)-celluloseether-chlorid (10 g) mit Imidazolium-N,N'-diyl-bis(2-hydroxy-3-propyl)-ether-chlorid von vernetzer Stärke (10 g) mit einer Austauschkapazität von 0,7 mmol/g verwendet wurde, das mit mikrokristalliner Cellulose (60 g; Korngröße 50 bis 300 μm) für pharmazeutische Zwecke gründlich vermischt wurde.

Das Imidazolium-N,N'-diyl-bis(2-hydroxy-3-propyl)-ether-chlorid von vernetzter Stärke wurde folgendermaßen hergestellt:

Zu einem Gemisch von 1,3-Bis(3-chlor-2-hydroxypropyl)-imidazoliniumsulfat (22 ml) (CS-B 229 063) und 3500 ml Wasser wurden 180 g Stärke unter Rühren zugegeben. Das Reaktionsgemisch wurde 30 min bei 20 °C gerührt, worauf 120 ml wässerige, 15 masse-%-ige Natriumhydroxidlösung zugegeben wurden und das Gemisch weitere 24 h bei 20 °C gerührt wurde. Das Reaktionsgemisch wurde dann mit 1 masse-%-iger Salzsäure neutralisiert; das Reaktionsprodukt wurde nach Filtrieren mit destilliertem Wasser bis zur negativen Reaktion auf Chlorid gewaschen. Anschließend wurde es mit Ethanol und Aceton gewaschen und bei 20 °C getrocknet. Schließlich wurde es auf eine Korngröße von 50 bis 300 μm gesiebt.

**Beispiel 15**

Es wurde wie in Beispiel 1 verfahren mit dem Unterschied, daß ein Gemisch von Diethylaminoethylcelluose (10 g) und Diethylaminohydroxypropylcellulose (10 g) mit einer Austauschkapazität von 0,7 mmol/g verwendet wurde. Das Gemisch wurde mit mikrokristalliner Cellulose (60 g, Korngröße 50 bis 300 μm) für pharmazeutische Zwecke gründlich vermischt.

**Beispiel 16**

Es wurde wie in Beispiel 1 verfahren mit dem Unterschied, daß ein Gemisch von trimethylammoniumpropyl-vernetzter Stärke (10 g) und diethylaminoethyl-vernetzter Stärke (10 g) mit einer Austauschkapazität von 0,7 mmol/g verwendet wurde. Das Gemisch wurde mit mikrokristalliner Cellulose (60 g, Korngröße 50 bis 300 μm) für pharmazeutische Zwecke gründlich vermischt.

### Beispiel 17 - Heparinabtrennung

Zu 0,5 bis 2 ml Blutplasma in einem Probeglas (12x90 mm) wurde eine Tablette des Präparats nach einem der Beispiele 1 bis 16 zugegeben und 1 bis 3 min bei 20 °C quellengelassen. Dann wurde der Probeglasinhalt mit einem Vibrator intensiv gemischt, bis die Tabletten im Plasma gleichmäßig zerfallen waren, worauf in einem Rührwerk 10 min bei 20 °C weiter gerührt wurde. Die Tablettensubstanz mit dem daran adsorbierten Heparin wurde in einer Laboratoriumszentrifuge (relative Zentrifugalbeschleunigung (RZB) 1200; 5 min) abzentrifugiert; entsprechende Plasmaaliquote wurden dann für Hämokoagulations-Tests verwendet, bei denen keinerlei Störungen auftraten.

Die Erfindung kann besonders in der Medizin und insbesondere in der Hämatologie bei Untersuchungen von mit Heparin behandelten Patienten auf das reale hämostatische Potential sowie überall vorteilhaft angewandt werden, wo eine einfache und rasche Abtrennung von Heparin aus Blut bzw. Blutplasma in vitro erforderlich ist.

### Ansprüche

1. Präparat zur Abtrennung von Heparin aus Blut und Blutplasma in vitro,
**gekennzeichnet durch**
mindestens ein blutunlösliches Stärkederivat mit daran gebundenen tertiären oder quartären Aminogruppen und/oder mindestens ein Cellulosederivat, das ausgewählt ist unter
Diethylaminoethylcellulose,
Dimethylaminoethylcellulose,
Trimethylammoniummethylcellulose,
Diethylaminopropylcellulose,
Triethylammoniumpropylcellulose,
Dimethylaminopropylcellulose,
Trimethylammoniumpropylcellulose,
Diethylamino-2-hydroxypropylcellulose,
Polyethylenimincelluose,
Triethylammonium-2-hydroxypropylcellulose,
Dimethylamino-2-hydroxypropylcellulose,
mit einem Gemisch von Epichlorhydrin und Diethylamin in alkalischen Medium vernetzter Cellulose,
Imidazolium-N,N'-diyl-bis(2-hydroxy-3-propyl)-celluloseether-chlorid,
Dimethylamino-bis(2-hydroxy-3-propyl)-celluloseether-chlorid und
Benzyldimethylammonium-2-hydroxypropylcellulose.

2. Präparat nach Anspruch 1, dadurch gekennzeichnet, daß die Austauschkapazität 0,3 bis 1,3 mmol/g beträgt.

3. Präparat nach Anspruch 1 oder 2, dadurch gekennzeichnet, daß das Cellulosederivat Imidazolium-N,N'-diyl-bis(2-hydroxy-3-propyl)-celluloseether-chlorid ist.

4. Präparat nach einem der Ansprüche 1 bis 3, dadurch gekennzeichnet, daß es bis zu 12 Masseteile mikrokristalline Cellulose pro Masseteil des Stärke-und/oder Cellulosederivats enthält.

5. Präparat nach einem der Ansprüche 1 bis 4, dadurch gekennzeichnet, daß das Stärkederivat ausgewählt ist unter:
diethylaminoethyl-vernetzter Stärke,
dimethylaminoethyl-vernetzter Stärke,
trimethylammoniumethyl-vernetzter Stärke,
diethylaminopropyl-vernetzter Stärke,
triethylammoniumpropyl-vernetzter Stärke,
dimethylaminopropyl-vernetzter Stärke,
trimethylammoniumpropyl-vernetzter Stärke,
diethylamino-2-hydroxypropyl-vernetzter Stärke,
polyethylenimin-vernetzter Stärke,
triethylammonium-2-hydroxypropyl-vernetzter Stärke,
dimethylamino-2-hydroxypropyl-vernetzter Stärke,
mit einem Gemisch von Epichlorhydrin und Diethylamin in alkalischem Medium vernetzter Stärke,
Imidazolium-N,N'-diyl-bis(2-hydroxy-3-propyl)-ether-chlorid von vernetzter Stärke,
Dimethylamino-bis(2-hydroxy-3-propyl)-ether-chlorid von vernetzter Stärke
und
benzyldimethylammonium-2-hydroxypropyl-vernetzter Stärke.

6. Präparat nach einem der Ansprüche 1 bis 5, dadurch gekennzeichnet, daß das Stärkederivat Imidazolium-N,N'-diyl-bis(2-hydroxy-3-propyl)-ether-chlorid von vernetzter Stärke ist.

7. Präparat nach einem der Ansprüche 1 bis 6, dadurch gekennzeichnet, daß das Stärke-bzw. Cellulosederivat aus Teilchen einer Teilchengröße von 50 bis 300 μm besteht.

8. Präparat nach einem der Ansprüche 1 bis 7, dadurch gekennzeichnet, daß es in Form von Tabletten vorliegt.

9. Verfahren zur Abtrennung von Heparin aus Blut und Blutplasma in vitro durch Inkontaktbringen des Bluts bzw. Blutplasma mit einem Sorbens für Heparin und Abtrennung des Bluts bzw. Blutplasmas vom Sorbens, dadurch gekennzeichnet, daß als Sorbens ein Präparat nach einem der Ansprüche 1 bis 8 verwendet wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 165 568 (INTERMEDICAT GMBH) * Seiten 12-14; Ansprüche 17-21, 23 * --- | 1,4,7 | G 01 N 33/86 // C 08 B 11/145 C 08 B 31/12 B 01 J 20/22 |
| A,D | US-A-4 226 599 (BUTLER et al.) * ganzes Dokument * --- | 1,2,4,8,9 | |
| A | CHEMICAL ABSTRACTS, Band 104, Nr. 13, 31. März 1986, Seite 340, Spalte 2, Zusammenfassungsnr. 125879r, Columbus, Ohio, US; A.M. CUMMING et al.: "In vitro neutralization of heparin in plasma prior to the activated partial thromboplastia time test: an assessment of four heparin antagonists and two anion exchange resins" --- | 1 | |
| A | DE-A-1 945 471 (MERCK PATENT GMBH) * Ansprüche 1-2 * ----- | 1 | |

**RECHERCHIERTE SACHGEBIETE (Int. Cl.4)**

G 01 N 33/00
C 12 Q 1/00
B 01 J 20/00
A 61 M 1/00
C 08 B 37/00
B 01 J 13/00
C 12 H 1/00

Der vorliegende Recherchenbericht wurde für alle Patentansprüche erstellt

| Recherchenort | Abschlußdatum der Recherche | Prüfer |
|---|---|---|
| BERLIN | 15-05-1988 | DE KOK A.J. |